# EUROPÄISCHE PATENTSCHRIFT

(11) **EP 0 754 764 B1**
(45) Veröffentlichungstag und Bekanntmachung des Hinweises auf die Patenterteilung: **09.04.2003**
(21) Anmeldenummer: 96111586.2
(22) Anmeldetag: 18.07.1996
(51) Int. Cl.: C12Q 1/68, C12P 19/34, C07H 21/04

(54) **Verfahren und Reagenz zur spezifischen Bestimmung von mRNA**
Method and reagent for the specific determination of mRNA
Procédé et réactif pour la détermination spécifique d'ARNm

(30) Priorität: 21.07.1995 DE 19526431
(43) Veröffentlichungstag der Anmeldung: 22.01.1997
(73) Patentinhaber: Roche Diagnostics GmbH, 68298 Mannheim (DE)
(72) Erfinder: Leying, Hermann, Dr., 83673 Bichl (DE); Hinzpeter, Matthias, Dr., 80689 München (DE); Fritton, Hans-Peter, Dr., 69509 Mörlenbach (DE); Wittor, Heiko, 82327 Tutzing (DE)

(56) Entgegenhaltungen:
- WO-A-90/06042
- WO-A-90/10716
- WO-A-93/15228
- REYES-ENGEL A. ET AL.: "Direct quantification of specific mRNA using a selected biotinylated oligonucleotide by free solution capillary electrophoresis" NUCLEIC ACIDS RESEARCH, Bd. 21, Nr. 3, 1993, GB, Seiten 759-760, XP002093315
- DATABASE WPI Week 9442 20.September 1994 Derwent Publications Ltd., London, GB; AN 94-337443 XP002093316 & JP 06 261 800 A (HITACHI LTD)
- K. CUDDY ET AL.: "RT-PCR with affinity-captured mRNA" NUCLEIC ACIDS RESEARCH, Bd. 21, Nr. 9, 1993, GB, Seite 2281 XP002093991

## Beschreibung

Die Erfindung betrifft ein Verfahren und Reagenz bzw. einen Kit zur spezifischen Bestimmung von mRNA, d.h. von Poly(A)-Sequenzen beinhalteten Nukleinsäuren an beschichteten Festphasen. Mit dem erfindungsgemäßen Verfahren können mRNA sowohl aus Mischungen von Gesamt-RNA als auch in Extrakten von Zellkulturen und Gewebezellen quantifiziert bzw. detektiert werden. Eine vorgeschaltete Anreicherung oder Isolierung der mRNA ist nicht erforderlich.

Fast alle in eukaryotischen Zellen vorhandenen mRNAs weisen eine endständige Sequenz von ungefähr 20 bis 250 Adenosin-Nukleotiden auf, was bei den meisten Reinigungsverfahren von mRNA ausgenutzt wird. Eine Standardmethode zur Detektion und Quantifizierung von mRNA stellt heute die kombinierte Anwendung von reverser Transkriptase-(RT-) und PCR-Reaktion (RT-PCR) dar (Larrick, J.W., Trends Biotechnol. 10, 146-152 (1992); Kawasaki, E.S., PCR Protocols: A Guide to Methods and Applications (eds. Innis, M.A. et al.), Academic Press, San Diego, California (1990)). Für diese Methode wird als Ausgangsmaterial gereinigte Gesamt-RNA (rRNA, tRNA, mRNA) oder gereinigte mRNA benötigt, da die bei der RT-PCR eingesetzten Enzyme, insbesondere Polymerasen durch Verunreinigungen sehr leicht inhibiert werden. Zur Reinigung von RNA bzw. mRNA sind eine Reihe von Methoden bekannt, wie beispielsweise die Extraktion mit organischen Lösungsmitteln (z.B. Phenol/Chloroform), die Reinigung über Oligo(dT)-Cellulose oder die Isolierung unter Verwendung von Oligo(dT)-beschichteten Magnetpartikeln (Sambrook, J., Fritsch, E.F. und T. Maniatis, Molecular Cloning: A Laboratory Manual, Cold Spring Harbor Laboratory Press, Cold Spring Harbor, N.Y., 2nd edition (1989); Farrell, R.E., RNA Modologies: A Laboratory Guide for Isolation and Characterization, Academic Press (1993)).

Diese Methoden sind somit umständlich und nur schlecht automatisierbar. Die Reinigung kann darüber hinaus durch Hybridisierung des Poly(A) tragenden 3'-Endes der eukaryotischen mRNA mit biotinyliertem Oligo(dT) erfolgen. Das Hybrid aus Biotin-Oligo(dT) und mRNA kann dann anschließend beispielsweise an Streptavidin beschichtete Magnetpartikel gebunden und isoliert werden. Dabei ist jedoch von Nachteil, daß lediglich größere Mengen an mRNA-Hybrid isoliert werden können und somit als Nachweisverfahren für spezifische mRNAs zu wenig empfindlich ist. Es wurde daraufhin eine RT-PCR an einer Festphase, d.h. an Magnetpartikeln entwickelt und beschrieben. Nachteilig bei diesem Verfahren ist jedoch, daß die Magnetpartikel nach Bindung und Waschen der mRNA in PCR-taugliche, d.h. insbesondere hitzebeständige Reaktionsgefäße überführt werden müssen, also ein weiterer Separationsschritt erforderlich ist.

Der Erfindung lag somit die Aufgabe zugrunde, ein verbessertes Verfahren zur Bestimmung von mRNA zur Verfügung zu stellen, wodurch die im Stand der Technik beschriebenen Nachteile überwunden werden.

Die Aufgabe wird gelöst durch ein Verfahren zur spezifischen Bestimmung von Poly(A)-Sequenzen enthaltenen Nukleinsäuren, dadurch gekennzeichnet, daß man
- geeignetes Probenmaterial mit einem Lysis- bzw. Hybridisierungspuffer versetzt und gegebenenfalls homogenisiert,
- die Probenlösung mit einer markierten Poly- oder Oligonukleotid-Probe oder eines markierten Nukleotidderivates, die mindestens eine zu Poly(A) komplementäre Sequenz tragen, oder einem für die zu bestimmende Nukleinsäure komplementären Oligo- oder Polynukleotid bzw. entsprechenden Derivaten in Kontakt bringt,
- ein Aliquot der Mischung in ein mit einer organisch chemischen Verbindung beschichtetes hitzestabiles, RNase-freies Reaktionsgefäß überführt und mindestens 10 Sekunden in einem Temperaturbereich von ca. 4° bis 50°C inkubiert,
- den Überstand aus dem Reaktionsgefäß entfernt und mehrere Male mit einer geeigneten Pufferlösung wäscht,
- mit einer Mischung, welche ein Enzym mit reverser Transkriptase(RT)-Aktivität, oder ein Enzym mit RT- und hitzestabiler DNA-Polymerase-Aktiviät enthält, vereinigt und bei ca. 30° bis 75°C inkubiert,
- gegebenenfalls die Mischung entfernt und mit Waschpuffer wäscht,
- eine weitere Mischung, die mindestens eine hitzestabile DNA-Polymerase und zwei spezifische DNA-Primer enthält, in das Reaktionsgefäß gibt und unter die Amplifikation fördernden Bedingungen inkubiert und
- die transkribierte und amplifizierte DNA enthaltende lösung entfernt und
- die in der Mischung enthaltene DNA gegebenenfalls auftrennt, insbesondere durch elektrophoretische Methoden und detektiert.

Besonders vorteilhaft ist, das bei dem erfindungsgemäßen Verfahren die Isolierung und Reinigung der mRNA aus entsprechendem Probenmaterial bereits in dem Gefäß erfolgt, das für die reverse Transkriptase (RT)-Reaktion und die anschließende PCR-Reaktion benutzt wird. D.h. eine sogenannte Probenvorbereitung (Isolierung und Reinigung) und Umschreibung bzw. Vervielfältigung (RT-PCR) erfolgen in ein und demselben Reaktionsgefäß.

Als zu analysierendes Probenmaterial kommen neben reinen mRNA-Fraktionen, insbesondere natürliche oder artifizielle Mischungen von Gesamt-RNA (rRNA, tRNA, mRNA), aber auch aus Zellkulturen gewonnene Fraktionen (Zelllysate) sowie Gewebezellextrakte bzw. Gewebehomogenate menschlichen oder tierischen Ursprungs sowie pflanzliche Extrakte in Betracht.

Geeignete Lysis- bzw. Hybridisierungspuffer basieren auf Puffersubstanzen, die eine gute Pufferkapazität zwischen einem pH-Wert-Bereich von ca. 5 bis 10, vorzugsweise zwischen pH 7,0 und 8,5 aufweisen. Entsprechende Puffersubstanzen sind beispielsweise Tris·HCl, HEPES, MOPS oder Tris·Borat.

Des weiteren können für das erfindungsgemäße Verfahren geeignete Puffer ein Disulfidreduzierendes Reagenz, wie z.B. Dithioerythritol, Dithiothreitol, Mercaptoethanol, vorzugsweise in einer Konzentration von 0,01 bis 1% (w/v) enthalten. Außerdem hat sich als vorteilhaft erwiesen, wenn das Puffersystem eine denaturierende Substanz, wie Deterengenzien in relativ hohen Konzentrationen enthält. Besonders geeignet haben sich hier Dodecylsulfatsalze oder entsprechende Abkömmlinge in einer Konzentration von 0,1 bis 15% (w/v) sowie, insbesondere bei Einsatz von RNase-reichem Gewebe, Salze vom Guanidiniumthiocyanat-Typ bzw. entsprechende Derivate und zwar in einem Konzentrationsbereich von ca. 1 bis 7 mM, insbesondere in einem Konzentrationsbereich von 1 bis 5 mM. Es hat sich dabei gezeigt, daß es von besonderem Vorteil ist, wenn die Lösungen für die Bestimmungen annähernd frei von RNase-Aktivität sind. Dies bedeutet, daß maximal 5% Restaktivität an RNase vorhanden sein dürfen.

Die für das erfindungsgemäße Verfahren einsetzbaren Puffersysteme können darüber hinaus weitere Salze, wie z.B. Lithiumchlorid oder sonstige Hilfsstoffe enthalten. Als vorteilhaft hat sich zudem erwiesen, wenn zusätzlich ein RNase-Inhibitor, wie z. B. ein aus Placenta gewonnener, zugegen ist und/oder die Pufferlösung zuvor sterilisiert bzw. mit Dimethyldicarbonat oder Diethylpyrocarbonat dekontaminiert wurde.

Als markierte Poly- bzw. Oligonukleotid-Probe oder markiertes Nukleotidderivat, die eine zu Poly(A) komplementäre Sequenz tragen, kommen insbesondere Oligo(dT)- oder Oligo(U)-Nukleotid-Probes oder entsprechende Peptidnukleinsäurederivate wie z.B. PNA, d.h. Nukleinsäuren mit Peptid-Backbone mit beliebiger Länge in Betracht. Vorzugsweise handelt es sich hierbei um 15 bis 30mere, oft ist ein 20meres ausreichend.

Als Markierungsgruppe für die Nukleotid-Probe kommen sämtliche an eine bestimmte Matrix koppel- bzw. bindbare Reste in Frage, wie z.B. Haptene, Proteine mit Antigen- bzw. Antikörperstruktur. Als besonders geeignet hat sich hier Biotin erwiesen, welches beispielsweise an die Oligo(dT)-Sequenz gebunden über Streptavidin oder Avidin an die Festphase koppelbar ist, für den Fall, daß es sich bei der organisch chemischen "Beschichtungssubstanz" um Streptavidin handelt. Alternativ kann es sich bei der Beschichtungssubstanz beispielsweise auch um Oligo(dT)-Nukleotide oder geeignete Chelat-Verbindungen handeln, die entweder durch Hybridisierung oder Komplexbildung zur Fixierung entsprechender Probes geeignet sind. Die Immobilisierung von mRNA an Streptavidin-beschichtete Festphasen bzw. Reaktionsgefäße stellt somit eine bequeme und zuverlässige Methode zur mRNA-Extraktion für die anschießende Transkription und Amplifikation dar. Darüber hinaus können auch andere Markierungsgruppen zur Fixierung an die Festphase verwendet werden. Hergestellt werden die Nukleotid-Probes wie beispielsweise Oligo(dT)-Probes nach bekannten Verfahren oder können von einschlägigen Anbietern bezogen werden.

Das polyadenylierte mRNA beinhaltende Probenmaterial wird mit der beispielsweise die markierte Oligo(dT)-Probe enthaltene wäßrige Lösung vereinigt. Der Hybridisierungsvorgang wird vorzugsweise bei ca. 37°C durchgeführt und ist in der Regel bereits nach wenigen Sekunden abgeschlossen. Ein Zeitraum von 10 Sekunden bis 10 Minuten, vorzugsweise 5 Minuten haben sich für die meisten Fälle als ausreichend für die Hybridisierung erwiesen.

Ein Aliquot der Biotin-Oliga(dT)-mRNA-Hybrid-Lösung wird anschließend direkt in das beschichtete, hitzestabile Reaktionsgefäß gegeben. Das Volumen des jeweiligen beschichteten Aliquots liegt optimalerweise in der Größenordnung des jeweiligen Gefäßvolumens und sollte nicht mehr als ca. Spmol des Hybrids enthalten. In der Regel ist eine Probe von ca. 20 bis 200 µl des Lysats ausreichend. Für die Immobilisierung an die beschichtete Festphase sind bereits wenige Sekunden, d.h. ca. 10 Sekunden Inkubationszeit bei ca. 4° bis 50°C, vorzugsweise bei ca. 37°C in den meisten Fällen ausreichend, sicherheitshalber wird der Vorgang in den meisten Fällen für eine bis 10 Minuten unterhalten.

Anschließend wird der Rest des Lysats entfernt und die gebundene RNA wird gründlich (ca. 5mal) gewaschen und der Waschpuffer anschließend quantitativ entfernt, so daß die reverse Transkriptase-Reaktion durchgeführt werden kann. Eine typische Mischung fiir die RT-Reaktion beinhaltet neben den üblichen Puffersubstanzen und Salzen, Magnesiumionen, alle vier Desoxynukleotidtriphosphate (dNTPs), ein Enzym mit RT-Aktivität oder ein Enzym mit RT- und hitzestabile DNA-Polymerase-Aktivität, wie beispielsweise DNA-Polymerase aus Thermus thermophilus (Tth) sowie vorteilhafterweise einen RNase-Inhibitor. Von einer solchen Mischung wird ein Aliquot von ca. 20 bis 200 µl in das Reaktionsgefäß gegeben. Als Enzym mit RT-Aktivität kommen insbesondere AMV- oder M-MuLV-RT, letztere insbesondere in ihrer RNaseH⁻ Form (US 5.244.797), zum Einsatz, vorzugsweise in einer Konzentration von 0,01 bis 5 U/µl. Vorzugsweise liegt die Endkonzentration der RT bei 0,1 bis 1,0 U/µl. Darüber hinaus hat sich gezeigt, daß auch RTs anderer Virusarten bzw. Quellen erfindungsgemäß verwendet werden können.

Die Inkubation für die cDNA-Synthese kann bei ca. 30° bis 75°C, vorzugsweise bei 35° bis 45°C durchgeführt werden und beträgt üblicherweise 30 bis 120 Minuten. Eine Inkubationstemperatur von ca. 42°C hat sich in vielen Fällen als besonders vorteilhaft erwiesen.

Nach Beendigung der Reaktion wird die Reaktionslösung entfernt und erneut gründlich mit Waschpuffer gewaschen. Anschließend wird ein bestimmtes Volumen eines PCR-Mixes in das beschichtete Reaktionsgefäß gegeben, in der Regel 20 bis 200 µl und unter wenig stringenten Bedingungen inkubiert. Ein optimaler PCR-Mix beinhaltet ein geeignetes Puffersystem, wie z.B. die in Maniatis et al. (2nd ed., 1989, s.o.), Seiten 14.15 bis 14.17 angegebenen Pufferkomponenten, Magnesiumionen in einem Konzentrationsbereich von ca. 0,5 bis 5,0 mM, eine bestimmte Menge einer hitzestabilen DNA-Polymerase, vorzugsweise zwischen 5 bis 300 mU/µl, alle vier dNTPs in jeweils einer Konzentration von 0,05 bis 0,6 mM, vorzugsweise jeweils ca. 0,2 mM und mindestens zwei DNA-Primer zwischen 0,1 und 1,0 pmol/µl, vorzugsweise jeweils ca. 0,4 pmol/µl.

Die PCR-Reaktion an sich erfolgt nach dem für den Fachmann bekannten Maßnahmen (sog. Standard-PCR), wie z.B. ein erster Zyklus bei 95°C (5 Min.), 50°C (2 Min.) und 72°C (3 Min.); die nachfolgenden Zyklen (bis zu insgesamt ca. 40 Zyklen) erfolgen dann nach folgendem Modus: 95°C (1 Min.), 50°C (2 Min.) und 72°C (3 Min. bzw. 10 Min. im letzten Durchlauf).

Nach Beendigung der PCR-Reaktion wird die die transkribierte und amplifizierte DNA enthaltene Lösung aus dem Reaktionsgefäß entfernt und beispielsweise elektrophoretisch aufgetrennt und, z.B. mittels Ethidiumbromid, detektiert.

Eine weitere vorteilhafte Ausführungsform ist, daß die RT- und PCR-Reaktion nicht sequentiell, sondern ohne zwischengeschalteten Waschschritt in einer gekoppelten Reaktion erfolgt. Bei einer solchen simultan verlaufenden Ausführungsform muß die RT-Lösung nach Beendigung der RT-Reaktion nicht entfernt werden. Eine besonders vorteilhafte Ausführungsform ist, wenn ein bifunktionelles Enzym, das sowohl RT- als auch DNA-Polymerase-Aktivität aufweist und hitzestabil ist, verwendet wird. Bei einer solchen sogenannten 1 Topf-/1 Puffer-Ausführungsform hat sich eine Inkubationszeit von ca. fünf Minuten bis eine Stunde als besonders vorteilhaft erwiesen.

Prinzipiell haben sich für das erfindungsgemäße Verfahren auch die in Maniatis et al., Molecular Cloning: A Laboratory Manual, Cold Spring Harbor Laboratory Press, Cold Spring Harbor, N.Y., 2nd edition (1989) beschriebenen Bedingungen für die RT- bzw. PCR-Reaktion als geeignet erwiesen.

Der Nachweis der erfindungsgemäß erhaltenen DNA-Mischung erfolgt in der Regel nach elektrophoretischer Auftrennung anhand geeigneter Hybridisierungsmethoden (Southem-Blot, ELISA). Es ist jedoch auch möglich, die DNA-Mischung ohne vorherige Auftrennung direkt zu detektieren. Entsprechende Methoden sind dem Fachmann bekannt. So konnte erfindungsgemäß noch mRNA in weniger als 1 Zelle detektiert werden.

Die Herstellung von beschichteten Festphasen bzw. Reaktionsgefäßen ist dem Fachmann bekannt (z.B. EP 0331127). Im Fall von Streptavidin- bzw. Avidin-beschichteten Trägermaterialien haben sich insbesondere die in EP 0344578 offenbarten Reaktionsgefäße als vorteilhaft erwiesen.

Ein weiterer Gegenstand der Erfindung ist ein Reagenz bzw. ein Kit zur Bestimmung von mRNA, der aus folgenden Komponenten besteht:
(a) Lysis- bzw. Hybridisierungspuffer,
(b) Pufferlösung mit markierter Nukleotid-Probe, die eine zu Poly(A) komplementäre Sequenz trägt,
(c) Waschpuffer,
(d) eine Mischung enthaltend ein Enzym mit reverser Transkriptase-Aktivität
(e) eine Mischung, die mindestens eine thermostabile DNA-Polymerase enthält und
(f) eine Vorrichtung mit mindestens einem beschichteten, RNase-freien Reaktionsgefäß

Als besonders bevorzugt hat sich für das erfindungsgemäße Verfahren erwiesen, wenn eine Oligo(dT)-Probe verwendet wird, die Biotin-markiert ist, und die für die Immobilisierung des Hybrids geeignete Festphase Streptavidin- bzw. Avidin-beschichtet ist. Des weiteren ist ein solcher Kit bevorzugt, der anstatt der zwei Mischungen (d) und (e) eine Mischung (d') umfaßt, die ein Enzym mit RT- und hitzestabiler DNA-Polymerase-Aktivität beinhaltet. Darüber hinaus gelten für den Kit die im Zusammenhang mit dem erfindungsgemäßen Verfahren offenbarten Merkmale.

Die folgenden Beispiele erläutern die Erfindung weiter:

### Beispiel 1

Die Streptavidin-beschichteten PCR-Gefäße dienen zur Durchführung der RT-PCR direkt aus Zellen ohne vorherige Isolierung der RNA.

Die Zellen werden in einem Lysis- bzw. Hybridisierungspuffer lysiert, die DNA geschert, die mRNA durch Hybridisierung des Poly A⁺ - Schwanz mit BidT₂₀ und dessen Bindung an die Streptavidinmatrix im Reaktionsgefäß immobilisiert. Über Reverse Transkriptase erfolgt die Synthese der entsprechenden cDNAs und über spezifische Primer mittels PCR die Amplifikation der spezifischen Transkripte mit anschließender Quantifizierung z.B. im Agarosegel über Ethidiumbromidfärbung.

In diesem Beispiel wird die Durchführung einer RT-PCR direkt aus humanen K562 Zellen zum Nachweis von mRNA von G3PDH beschrieben.

Alle verwendeten Komponenten stammen von Boehringer Mannheim GmbH, mit Ausnahme der PCR-Primer für humane G3PDH-mRNA (983 bp), die von Clontech stammen (Art. No. 5406-1). Die Puffer (Lysis-/Hybridisierungspuffer) sowie die RT- und PCR-Mischungen wurden wie folgt hergestellt:
1) Lysis- /Hybridisierungspuffer
   a) Detergenzpuffer (Volumen zur Herstellung von 250 ml):
      25,0 ml Tris·HCl, IM, pH 7,5 (4°C)
      15,0 ml LiCl, 5M
      5,0 ml EDTA, 0,5 M, pH 8,0
      25,0 ml Lithium-dodecylsulfat, 10% (w/v)
      193,0 mg 1,4-Dithioerythritol
      180,0 ml redist. Wasser (Die autoklavierten Stammlösungen sind bei RT stabil)
   b) GTC-Puffer
      4,0 M Guanidiniumthiocyanat (GTC)
      0,1 M Tris·HCl, pH 8,0
      1,0% 1,4-Dithiothreitol (w/v)
      0,5% Lauroylsarcosin (w/v) (bei -20°C lagerbar)
2) Biotin-markierte Oligo(dT)-Probe (Volumen für 20 µl)
   19,0 µl redist. Wasser
   1,0 µl Biotin-Oligo(dT)₂₀-Probe (^ 100 pmol)
3) Waschlösung (Volumen für 250 ml)
   2,5 ml Tris·HCl, 1 M, pH 78,5 (4°C)
   10,0 ml LiCl, 5 M
   0,5 ml EDTA, 0,5 M, pH 8,0
   237,0 ml redist. Wasser
4) RT-Mix (Volumen für 1 ml)
   204,1 µl Tris·HCl, 10 mM, pH 7,4
   51,0 µl Tris·HCl, 1 M, pH 8,3 (42°C)
   142,9 µl KCl, 1 M
   40,8 µl MgCl₂, 250 mM
   102,0 µl dNTPs, je 10 mM
   40,8 µl 1,4-Dithioerythritol, 100 mM
   20,0 µl RNase-Inhibitor (40 U/µl)
   398,4 µl redist. Wasser
   32,0 µl AMV reverse Transkriptase (25 U/µl)
5) PCR-Mix (Volumen für 1 ml)
   100,0 µl Taq-Puffer (10x)
   20,0 µl dNTPs, je 10 mM
   4,0 µl Primer, jew. (100 pmol/µl)
   867,0 µl redist. Wasser
   5,0 µl Taq Polymerase (5 U/µl)

Sämtliche Lösungen bzw. das für deren Herstellung verwendete redestillierte Wasser sollte zur Zerstörung eventuell vorhandener RNasen beispielsweise mit Dimethyldicarbonat (DMDC) oder Diethylpyrocarbonat (DEPC) behandelt werden.

Zelllyse und Immobilisieren der mRNA im SA-beschichteten Reaktionsgefäß K562 Zellen befanden sich im logarithmischen Wachstum mit 3x10⁵ Zellen/ml. 1 ml Zellsuspension wurde bei 300xg 5 Minuten abzentrifugiert, 1mal mit eiskaltem PBS gewaschen und das Pellet in flüssigem Stickstoff aufbewahrt. Das gefrorene Pellet wurde in 200 µl Lysepuffer resuspendiert, die DNA durch sechsmaliges Aufziehen mit einer 0,8 mm Kanüle geschert und aus dem Lysat durch Überpipettieren von je 20 µl auf vorgelegte 180 µl Lysepuffer eine log₁₀-Verdünnungsreihe angefertigt. In die Ansätze wurde je 5 pmol BidT₂₀ pipettiert und 5 Minuten bei 37°C hybridisiert. Je 50 µl der Ansätze wurden in Streptavidin-beschichteten PCR-Gefäßen pipettiert und 3 Minuten bei 37°C gebunden. Anschließend wurde die Lösung abpipettiert und die Gefäße auf Eis 4mal mit je 250 µl Waschpuffer gewaschen.

### cDNA Synthese

In die einzelnen Gefäßplätze wurde je 50 µl 1mal RT-Mix einpipettiert und ca. 2 Stunden bei 42°C inkubiert. Als Kontrolle wurde ein Gefäßplatz mit der höchsten RNA-Konzentration mit RT Mix ohne Reverse Transkriptase versehen, als weitere Kontrolle wurde ein Gefäßplatz ohne RNA mit RT Mix mit RT versehen. Anschließend folgt Hitzedenaturierung der RT während 10 Minuten, bei 65°C und wurde 4 mal mit je 250 µl Waschpuffer gewaschen.

### Amplifikation durch PCR

In die einzelnen Gefäßplätze wurde je 50 µl 1mal PCR-Mix einpipettiert und im Thermocycler die PCR-Läufe entsprechend den Angaben des Herstellers durchgeführt. Die PCR-Produkte wurden im 2% Agarosegel (mit Ethidiumbromid) mit 100 V 35 Minuten aufgetrennt und auf dem Schirm analysiert.

### Beispiel 2

Entsprechend den Angaben und Lösungen von Beispiel 1 wurde β-Actin-mRNA bestimmt. Im Ergebnis konnte in weniger als einer Zelle β-Actin-mRNA detektiert werden.

### Abbildung 1:

Ergebnis von Beispiel 1, wobei die Spuren 1 bis 7 des Elektrophoresegels folgende Proben widergeben:
- Spur 1:: Kontrolle mit Zellen, ohne RT
- Spur 2-5:: Verdünnungsreihe der Zelllysate nach erfolgtem erfindungsgemäßen Verfahren (bei Spur 4 im Original ist eine Bande sichtbar), folgende Mengen wurden aufgetragen: Spur 2: 7500 Zellen, Spur 3: 750 Zellen, Spur 4: 75 Zellen und Spur 5: 7,5 Zellen.
- Spur 6:: Kontrolle ohne Zellen, mit 1 x RT-Mix
- Spur 7:: DNA Molekulargewichtsstandard Nr. 4 (Boehringer Mannheim)

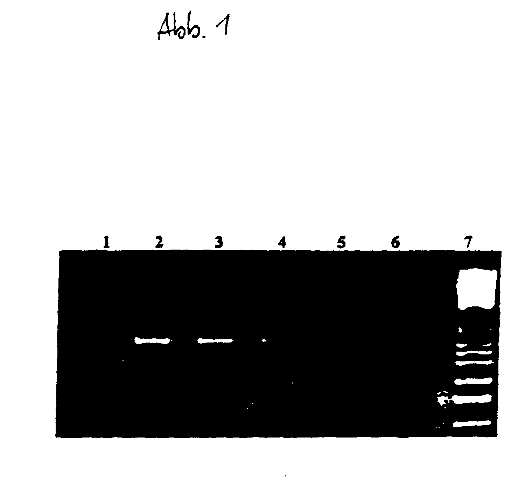

## Patentansprüche

1. Verfahren zur spezifischen Bestimmung von Poly(A)-Sequenzen enthaltenen Nukleinsäuren, **dadurch gekennzeichnet, dass** man
- geeignetes Probenmaterial mit einem Lysis- bzw. Hybridisierungspuffer versetzt und gegebenenfalls homogenisiert,
- die Probenlösung mit einer markierten Poly- oder Oligonukleotid-Probe oder eines markierten Nukleotidderivats, die mindestens eine zu Poly(A) komplementäre Sequenz tragen, oder einem für die zu bestimmende Nukleinsäure komplementären Oligo- oder Polynukleotid bzw. entsprechenden Derivaten in Kontakt bringt,
- ein Aliquot der Mischung in ein mit einer organisch chemischen Verbindung beschichtetes hitzestabiles, RNase-freies und zur Fixierung der Poly- oder Oligonukleotidprobe geeignetes Reaktionsgefäß überführt und mindestens 10 Sekunden in einem Temperaturbereich von ca. 4° bis 50°C inkubiert,
- den Überstand aus dem Reaktionsgefäß entfernt und mehrere Male mit einer geeigneten Pufferlösung wäscht,
- mit einer Mischung, welche ein Enzym mit reverser Transkriptase(RT)-Aktivität, enthält, vereinigt und bei ca. 30° bis 75°C inkubiert,
- gegebenenfalls die Mischung entfernt und mit Waschpuffer wäscht,
- eine weitere Mischung, die mindestens eine hitzestabile DNA-Polymerase und zwei spezifische unmarkierte DNA-Primer enthält, in das Reaktionsgefäß gibt und unter die Amplifikation fördernden Bedingungen inkubiert,
- die transkribierte und amplifizierte DNA enthaltende Lösung entfernt und
- die in der Mischung enthaltene DNA gegebenenfalls auftrennt, insbesondere durch elektrophoretische Methoden und detektiert.

2. Verfahren zur spezifischen Bestimmung von Poly(A)-Sequenzen enthaltenen Nukleinsäuren, **dadurch gekennzeichnet, dass** man
- geeignetes Probenmaterial mit einem Lysis- bzw. Hybridisierungspuffer versetzt und gegebenenfalls homogenisiert,
- die Probenlösung mit einer markierten Poly- oder Oligonukleotid-Probe oder eines markierten Nukleotidderivats, die mindestens eine zu Poly(A) komplementäre Sequenz tragen, oder einem für die zu bestimmende Nukleinsäure komplementären Oligo- oder Polynukleotid bzw. entsprechenden Derivaten in Kontakt bringt,
- ein Aliquot der Mischung in ein mit einer organisch chemischen Verbindung beschichtetes hitzestabiles, RNase-freies und zur Fixierung der Poly- oder Oligonukleotidprobe geeignetes Reaktionsgefäß überführt und mindestens 10 Sekunden in einem Temperaturbereich von ca. 4° bis 50°C inkubiert,
- den Überstand aus dem Reaktionsgefäß entfernt und mehrere Male mit einer geeigneten Pufferlösung wäscht,
- mit einer Mischung, welche ein Enzym mit RT- und hitzestabiler DNA-Polymerase-Aktivität und zwei spezifische unmarkierte DNA-Primer enthält, vereinigt und bei ca. 30° bis 75°C inkubiert,
- unter die Amplifikation fördernden Bedingungen inkubiert und
- die transkribierte und amplifizierte DNA enthaltene Lösung entfernt
- die in der Mischung enthaltene DNA gegebenenfalls auftrennt, insbesondere durch elektrophoretische Methoden und detektiert.

3. Verfahren nach Anspruch 1 oder 2, **dadurch gekennzeichnet, dass** als Probenmaterial eine Mischung bestehend aus Gesamt-RNA, kultivierten Zellen, Gewebezellen tierischen oder humanen Ursprungs oder planzliche Extrakte verwendet werden.

4. Verfahren nach Anspruch 1 - 3, **dadurch gekennzeichnet, dass** der Lysis- bzw. Hybridisierungspuffer eine Disulfid-Gruppen reduzierende Substanz, eine zwischen pH 7,0 und pH 8,5 puffernde Substanz und ein Detergenz oder denaturierendes Mittel sowie gegebenenfalls einen RNase-Inhibitor enthält.

5. Verfahren nach Anspruch 4, **dadurch gekennzeichnet, dass** der Puffer 0,5 bis 15,0% (w/v) eines Dodecylsulfatsalzes oder 1 bis 7 mM Guanidinumthiocyanat enthält.

6. Verfahren nach einem der vorangegangenen Ansprüche, **dadurch gekennzeichnet, dass** die Nukleotid-Probe mit Biotin markiert und das Reaktionsgefäß mit Streptavidin oder Avidin beschichtet ist.

7. Verfahren nach einem der vorangegangenen Ansprüche, **dadurch gekennzeichnet, dass** als markierte Nukleotid-Probe ein Oligo(dT)- oder ein Oligo(U)- oder ein PNA-Derivat verwendet wird.

8. Verfahren nach einem der vorangegangenen Ansprüche, **dadurch gekennzeichnet, dass** ein 15- bis 30meres Nukleotid-Probe verwendet wird und/oder die Hybridisierung innerhalb von einer bis 10 Minuten bei ca. 37°C durchgeführt wird.

9. Verfahren nach einem der Ansprüche 6 - 8, **dadurch gekennzeichnet, dass** ein Aliquot, welches 5 pmol Poly(A/dT)-Hybrid oder weniger enthält, in ein Streptavidin-beschichtetes Reaktionsgefäß der entsprechenden Größenordnung gegeben wird.

10. Verfahren nach einem der vorangegangenen Ansprüche, **dadurch gekennzeichnet, dass** die Mischung für die reverse Transkription 0,01 bis 5,0 U/µl AMV- oder M-MuLV- reverse Transkriptase, ein SH-Reagenz, einen RNase-Inhibitor, Kalium- und Magnesiumionen sowie eine geeignete Puffersubstanz enthält

11. Verfahren nach Anspruch 1, 2 oder 10 **dadurch gekennzeichnet, dass** die RT-Reaktion bei ca. 35° bis 45°C durchgeführt wird.

12. Verfahren nach einem der vorangegangenen Ansprüche, **dadurch gekennzeichnet, dass** die PCR-Reaktion in Gegenwart von 5 bis 300 mU/µl einer hitzestabilen DNA-Polymerase, jeweils ca. 0,1 bis 1,0 pmol/µl der spezifischen Primer, ca. 0,05 bis 0,6 mM der dNTPs, eines geeigneten PCR-Puffersystems und gegebenenfalls in Gegenwart von Magnesiumionen stattfindet.

13. Verfahren nach einem der Ansprüche 1 bis 9, **dadurch gekennzeichnet, dass** man ein Enzym, welches RT- und DNA-Polymerase-Aktivität besitzt und hitzestabil ist, verwendet wird und ca. fünf Minuten bis eine Stunde unter für die PCR-Reaktion günstigen Bedingungen inkubiert.

14. Kit für die Bestimmung von mRNA bestehend aus folgenden Komponenten:
(a) Lysis- bzw. Hybridisierungspuffer,
(b) Pufferlösung mit markierter Nukleotid-Probe bzw. entsprechendem Derivat, die mindestens eine zu Poly(A) komplementäre Sequenz trägt,
(c) Waschpuffer,
(d) eine Mischung enthaltend ein Enzym mit reverser Transkriptase-Aktivität,
(e) eine Mischung, die mindestens eine thermostabile DNA-Polymerase, sowie unmarkierte Primer enthält, sowie
(f) eine Vorrichtung mit mindestens einem beschichtetem hitzestabilen, RNasefreiem Reaktionsgefäß.

15. Kit nach Anspruch 14, **dadurch gekennzeichnet, dass** eine Enzym-Mischung enthaltend ein Enzym mit RT- und thermostabiler DNA-Polymerase-Aktivität verwendet wird.

16. Kit nach Anspruch 14 - 15, **dadurch gekennzeichnet, dass** eine Oligo(dT)-Probe mit Biotin markiert verwendet wird und das Reaktionsgefäß mit Streptavidin oder Avidin beschichtet ist.

## Claims

1. Method for the specific determination of nucleic acids containing poly(A) sequences, **characterized in that**
- a lysis or hybridization buffer is added to a suitable sample material and optionally homogenized,
- the sample solution is brought into contact with a labelled polynucleotide or oligonucleotide probe or a labelled nucleotide derivative which carries at least one sequence complementary to poly(A) or with an oligonucleotide or polynucleotide or a corresponding derivative that is complementary to the nucleic acid to be determined,
- an aliquot of the mixture is transferred into a heat-stable, RNase-free reaction vessel which is coated with an organic chemical compound and is suitable for immobilizing the polynucleotide or oligonucleotide probe and it is incubated for at least 10 seconds in a temperature range of about 4° to 50°C,
- the supernatant is removed from the reaction vessel and washed several times with a suitable buffer solution,
- it is combined with a mixture which contains an enzyme with reverse transcriptase (RT) activity and incubated at ca. 30° to 75°C,
- if required, the mixture is removed and it is washed with washing buffer,
- another mixture which contains a heat-stable DNA polymerase and two specific unlabelled DNA primers is added to the reaction vessel and incubated under conditions that support amplification,
- the solution containing the transcribed and amplified DNA is removed and
- the DNA present in the mixture is optionally separated especially by means of electrophoretic methods and detected.

2. Method for the specific determination of nucleic acids containing poly(A) sequences, **characterized in that**
- a lysis or hybridization buffer is added to a suitable sample material and optionally homogenized,
- the sample solution is brought into contact with a labelled polynucleotide or oligonucleotide probe or a labelled nucleotide derivative which carries at least one sequence complementary to poly(A) or with an oligonucleotide or polynucleotide or a corresponding derivative that is complementary to the nucleic acid to be determined,
- an aliquot of the mixture is transferred into a heat-stable, RNase-free reaction vessel which is coated with an organic chemical compound and is suitable for immobilizing the polynucleotide or oligonucleotide probe and it is incubated for at least 10 seconds in a temperature range of about 4° to 50°C,
- the supernatant is removed from the reaction vessel and washed several times with a suitable buffer solution,
- it is combined with a mixture which contains an enzyme with RT and heat-stable DNA polymerase activity and two specific unlabelled DNA primers and incubated at ca. 30° to 75°C,
- incubated under conditions which support amplification and,
- the solution containing the transcribed and amplified DNA is removed and
- the DNA present in the mixture is optionally separated especially by means of electrophoretic methods and detected.

3. Method as claimed in claim 1 or 2, **characterized in that** a mixture consisting of total RNA, cultured cells, tissue cells of animal or human origin or plant extracts are used as the sample material.

4. Method as claimed in claims 1 - 3, **characterized in that** the lysis or hybridization buffer contains a substance that reduces disulphide groups, a substance buffering between pH 7.0 and pH 8.5 and a detergent or denaturing agent and optionally an RNase inhibitor.

5. Method as claimed in claim 4, **characterized in that** the buffer contains 0.5 to 15.0 % (w/v) of a dodecyl sulphate salt or 1 to 7 mM guanidinium thiocyanate.

6. Method as claimed in one of the previous claims, **characterized in that** the nucleotide probe is labelled with biotin and the reaction vessel is coated with streptavidin or avidin.

7. Method as claimed in one of the previous claims, **characterized in that** an oligo(dT) or an oligo(U) or a PNA derivative is used as the labelled nucleotide probe.

8. Method as claimed in one of the previous claims 6 - 8, **characterized in that** a 15mer to 30mer nucleotide probe is used and/or the hybridization is carried out within one to 10 minutes at ca. 37°C.

9. Method as claimed in one of the previous claims, **characterized in that** an aliquot which contains 5 pmol poly(A/dT) hybrid or less is added to a streptavidin-coated reaction vessel of the corresponding size.

10. Method as claimed in one of the previous claims, **characterized in that** the mixture for the reverse transcription contains 0.01 to 5.0 U/µl AMV or M-MuLV transcriptase, an SH reagent, an RNase inhibitor, potassium and magnesium ions and a suitable buffer substance.

11. Method as claimed in claim 1, 2 or 10, **characterized in that** the RT reaction is carried out at ca. 35° to 45°C.

12. Method as claimed in one of the previous claims, **characterized in that** the PCR reaction takes place in the presence of 5 to 300 mU/µl of a heat-stable DNA polymerase, ca. 0.1 to 1.0 pmol/µl of each of the specific primers, ca. 0.05 to 0.6 mM of the dNTPs, a suitable PCR buffer system and optionally in the presence of magnesium ions.

13. Method as claimed in one of the claims 1 to 9, **characterized in that** an enzyme which has RT and DNA polymerase activity and is heat-stable is used and is incubated for about five minutes to one hour under conditions that are favourable for the PCR reaction.

14. Kit for determining mRNA comprising the following components:
(a) lysis or hybridization buffer,
(b) buffer solution containing a labelled nucleotide probe or a corresponding derivative which carries at least one sequence that is complementary to poly(A),
(c) washing buffer,
(d) a mixture containing an enzyme with reverse transcriptase activity,
(e) a mixture which contains at least one thermostable DNA polymerase and unlabelled primers, and
(f) a device containing at least one coated heat-stable, RNase-free reaction vessel.

15. Kit as claimed in claim 14, **characterized in that** an enzyme mixture containing an enzyme having RT and thermostable DNA polymerase activity is used.

16. Kit as claimed in claim 14 - 15, **characterized in that** an oligo(dT) probe labelled with biotin is used and the reaction vessel is coated with streptavidin or avidin.

## Revendications

1. Procédé pour la détermination spécifique d'acides nucléiques contenant des séquences poly(A), **caractérisé**
- **en ce qu'**on additionne un matériau d'échantillon approprié d'un tampon de lyse ou d'hybridation et qu'on homogénéise le cas échéant,
- **en ce qu'**on met la solution d'échantillon en contact avec une sonde marquée de polynucléotide ou d'oligonucléotide ou un dérivé marqué de nucléotide qui portent au moins une séquence complémentaire du poly(A) ou un oligonucléotide ou un polynucléotide ou des dérivés correspondants complémentaires de l'acide nucléique à déterminer,
- **en ce qu'**on transfère une partie aliquote du mélange dans un récipient de réaction revêtu d'un composé chimique organique, stable à la chaleur, exempt d'ARNase et approprié pour la fixation de la sonde de polynucléotide ou d'oligonucléotide et qu'on incube au moins pendant 10 secondes dans une plage de température d'environ 4°C à 50°C,
- **en ce qu'**on élimine la phase surnageante du récipient de réaction et qu'on lave plusieurs fois avec une solution tampon appropriée,
- **en ce qu'**on rassemble avec un mélange qui contient une enzyme présentant une activité de transcriptase inverse (TI) et qu'on incube à environ 30°C jusqu'à 75°C,
- **en ce qu'**on élimine le cas échéant le mélange et qu'on lave avec un tampon de lavage,
- **en ce qu'**on introduit dans le récipient de réaction un autre mélange contenant au moins une ADN-polymérase stable à la chaleur et deux amorces d'ADN spécifiques non marquées et qu'on incube sous des conditions favorisant l'amplification,
- **en ce qu'**on élimine la solution contenant l'ADN transcrit et amplifié et
- **en ce qu'**on sépare le cas échéant l'ADN contenu dans le mélange, en particulier par des procédés électrophorétiques et qu'on le détecte.

2. Procédé pour la détermination spécifique d'acides nucléiques contenant des séquences poly(A) **caractérisé**
- **en ce qu'**on additionne un matériau d'échantillon approprié d'un tampon de lyse ou d'hybridation et qu'on homogénéise le cas échéant,
- **en ce qu'**on met la solution d'échantillon en contact avec une sonde marquée de polynucléotide ou d'oligonucléotide ou un dérivé marqué de nucléotide qui portent au moins une séquence complémentaire du poly(A) ou un oligonucléotide ou un polynucléotide ou des dérivés correspondants complémentaires de l'acide nucléique à déterminer,
- **en ce qu'**on transfère une partie aliquote du mélange dans un récipient de réaction revêtu d'un composé chimique organique, stable à la chaleur, exempt d'ARNase et approprié pour la fixation d'une sonde de polynucléotide ou d'oligonucléotide et qu'on incube au moins pendant 10 secondes dans une plage de température d'environ 4°C à 50°C,
- **en ce qu'**on élimine la phase surnageante du récipient de réaction et qu'on lave plusieurs fois avec une solution tampon appropriée,
- **en ce qu'**on rassemble avec un mélange qui contient une enzyme présentant une activité Tl et une activité ADN-polymérase stable à la chaleur et deux amorces d'ADN spécifiques non marquées et qu'on incube à environ 30°C jusqu'à 75°C,
- **en ce qu'**on incube sous des conditions favorisant l'amplification,
- **en ce qu'**on élimine la solution contenant l'ADN transcrit et amplifié et
- **en ce qu'**on sépare le cas échéant l'ADN contenu dans le mélange, en particulier par des procédés électrophorétiques et qu'on le détecte.

3. Procédé selon la revendication 1 ou 2, **caractérisé en ce qu'**on utilise comme matériau d'échantillon un mélange constitué de l'ARN total, des cellules cultivées, des cellules de tissus d'origine animale ou humaine ou des extraits végétaux.

4. Procédé selon la revendication 1 à 3, **caractérisé en ce que** le tampon de lyse ou d'hybridation contient une substance réduisant les groupes disulfure, une substance tampon réglant le pH entre 7,0 et 8,5 et un détergent ou un agent de dénaturation ainsi que, le cas échéant, un inhibiteur d'ARNase.

5. Procédé selon la revendication 4, **caractérisé en ce que** le tampon contient 0,5 à 15,0 % (P/V) d'un sel de sulfate de dodécyle ou 1 à 7 mM de thiocyanate de guanidinum.

6. Procédé selon l'une quelconque des revendications précédentes, **caractérisé en ce que** la sonde de nucléotide est marquée avec de la biotine et que le récipient de réaction est revêtu de streptavidine ou d'avidine.

7. Procédé selon l'une quelconque des revendications précédentes, **caractérisé en ce qu'**on utilise comme sonde de nucléotide marquée un dérivé oligo(dT) ou oligo (U) ou PNA.

8. Procédé selon l'une quelconque des revendications précédentes, **caractérisé en ce qu'**on utilise une sonde de nucléotide 15-mère à 30-mère et/ou que l'hybridation est réalisée en un laps de temps de 1 à 10 minutes à environ 37°C.

9. Procédé selon l'une quelconque des revendications 6 à 8, **caractérisé en ce qu'**une partie aliquote, qui contient 5 pmoles d'hybride poly(A/dT) ou moins est introduite dans un récipient de réaction revêtu de streptavidine d'un ordre de grandeur correspondant.

10. Procédé selon l'une quelconque des revendications précédentes, **caractérisé en ce que** le mélange pour la transcription inverse contient 0,01 à 5,0 U/µl de transcriptase inverse AMV ou M-MuLV, un réactif SH, un inhibiteur d'ARNase, des ions de potassium et de magnésium ainsi qu'une substance tampon appropriée.

11. Procédé selon Tune quelconque des revendications 1, 2 ou 10, **caractérisé en ce que** la réaction Tl est réalisée à environ 35°C jusqu'à 45°C.

12. Procédé selon Tune quelconque des revendications précédentes, **caractérisé en ce que** la réaction de PCR a lieu en présence de 5 à 300 mU/µl d'une ADN-polymérase stable à la chaleur, à chaque fois environ 0,1 à 1,0 pmole/µl de l'amorce spécifique, environ 0,05 à 0,6 mM du dNTP, un système tampon approprié pour la PCR et, le cas échéant, en présence d'ions de magnésium.

13. Procédé selon Tune quelconque des revendications 1 à 9, **caractérisé en ce qu'**on utilise une enzyme qui présente une activité Tl et ADN-polymérase et qui est stable à la chaleur, et qu'on incube environ cinq minutes à une heure sous des conditions favorables pour la PCR.

14. Kit pour la détermination d'ARNm constitué des composants suivants :
(a) un tampon de lyse ou d'hybridation,
(b) une solution tampon contenant une sonde de nucléotide marquée, ou un dérivé correspondant, qui porte au moins une séquence complémentaire du poly(A),
(c) un tampon de lavage,
(d) un mélange contenant une enzyme présentant une activité de transcriptase inverse,
(e) un mélange qui contient au moins une ADN-polymérase stable à la chaleur, ainsi qu'une amorce non marquée ainsi que
(f) un dispositif présentant au moins un récipient de réaction revêtu, stable à la chaleur, exempt d'ARNase.

15. Kit salon la revendication 14, **caractérisé en ce qu'**on utilise un mélange d'enzymes contenant une enzyme présentant une activité TI et une activité ADN-polymérase stable à la chaleur.

16. Kit selon la revendication 14 et 15, **caractérisé en ce qu'**on utilise un échantillon oligo(dT) marqué à la biotine et que le récipient de réaction est revêtu de streptavidine ou d'avidine.
